# EUROPÄISCHE PATENTSCHRIFT

(11) **EP 2 178 495 B1**
(45) Veröffentlichungstag und Bekanntmachung des Hinweises auf die Patenterteilung: **25.06.2014**
(21) Anmeldenummer: 08787221.4
(22) Anmeldetag: 14.08.2008
(51) Int. Cl.: A61K 8/44, A61K 8/49, A61Q 5/08

(54) **REDUKTIVE ENTFÄRBUNG KERATINHALTIGER FASERN**
REDUCTIVE DECOLOURATION OF KERATIN-CONTAINING FIBRES
DÉCOLORATION DE FIBRES KÉRATINIQUES PAR RÉDUCTION

(30) Priorität: 23.08.2007 DE 102007039954
(43) Veröffentlichungstag der Anmeldung: 28.04.2010
(73) Patentinhaber: Henkel AG & Co. KGaA, 40589 Düsseldorf (DE)
(72) Erfinder: EMMERLING, Winfried, 25436 Tornesch (DE); BARTELS, Holger, 20148 Hamburg (DE); NEUBUESER, Inge, 22587 Hamburg (DE)
(86) Internationale Anmeldenummer: PCT/EP2008/060685
(87) Internationale Veröffentlichungsnummer: WO 2009/024525

(56) Entgegenhaltungen:
- WO-A-2008/012733
- US-A- 5 516 507
- US-A- 6 042 845
- US-A1- 2005 123 487
- US-B1- 6 743 419

## Beschreibung

Die Anmeldung betrifft Mittel, enthaltend eine Kombination aus (a) mindestens einer organischen Verbindung, die mindestens eine Thiolgruppe und mindestens eine gegebenenfalls derivatisierte Carboxylgruppe trägt und (b) mindestens einer organischen Verbindung, ausgewählt aus der Gruppe, die gebildet wird aus cyclischen, organischen Carbonaten, die Verwendung dieser Mittel für die Entfärbung karatinhaltiger Fasern, beispielsweise menschlicher Haare, und ein Verfahren zur Entfärbung keratinhaltiger Fasern sind Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Zuerst wurden natürliche Farbstoffe, wie z.B. Purpur oder Carmin, verwendet. Durch den rasanten Fortschritt der Wissenschaften wurden über die Jahre für jede Anwendung maßgeschneiderte, synthetische Farbstoffe zugänglich. Für das Färben, beispielsweise von Papier, Textilien oder keratinhaltigen Fasern, kommen im allgemeinen entweder direktziehende Farbstoffe oder Oxidationsfarbstoffe In Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hineln diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Amino-gruppe, Diaminopyridinderivate, heterocyclische Hydrazone, 4-Aminopyrazolonderivate sowie 2,4,5,6-Tetraaminopyrimidin und dessen Derivate eingesetzt

Spezielle Vertreter sind beispielsweise p-Phenylandiamin, p-Toluylendiamin, 2,4,5,6- Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-_{hydroxypyrimidin} und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch synthetische Färbung aufgetragenen Farbstoffe reduktiv ein. Es tritt somit kaum eine Aufhellung des Haars auf.

Die Druckschrift EP-A1-943 316 betrifft die Verwendung von Thiolgruppenhaltigen Verbindungen in Kombination mit alpha-Ketocarbonsäuren in Mitteln zur Entfärbung gefärbten Haars.

Die Druckschrift DE-A-102004045353 hat die Entfärbung gefärbter Haare unter Zuhilfenahme von Glyzerin oder Derivaten davon, organischen Carbonaten oder von Fettsäuredimethylamiden zum Gegenstand.

Allerdings bedürfen die Entfärbemittel des Standes der Technik einer Verbesserung der Entfärbeleistung und einer Verkürzung der Anwendungszeit.

Aufgabe der vorliegenden Erfindung ist es, reduktive Entfärbemittel bereitzustellen, die das Substrat dauerhaft ohne Nachdunkelung entfärben. Die Substratstruktur soll dabei geschont werden. Ferner sollten die in den Entfärbemilteln eingesetzten Reduktionsmittel für eine kosmetische Verwendung physiologisch verträglich und toxikologisch unbedenklich sein. Die Einwirkzeit der Entfärbemittel soll möglichst gering sein.

Überraschenderweise eignen sich die erfindungsgemäßen Mittel bzw. die darin enthaltene Wirkstoffkombination hervorragend zur beschleuniglen und verbesserten Entfärbung gefärbter Substrate, wie beispielsweise Papier, Textilien oder keratinhaltigen Fasern, insbesondere menschlichem Haar. Die erfindungsgemäße Wirkstoffkombination eignet sich besonders zur faserschonenden Entfärbung keratinhaltiger Fasern.

Unter keratinhaltigen Fasern sind beispielsweise Wolle, Pelze, Federn und insbesondere menschliche Haare zu verstehen.

Ein erster Gegenstand der Erfindung ist daher die Verwendung eines Mittels zur reduktiven Entfärbung keratinhaltiger Fasern, insbesondere menschlicher Haare, mit einem pH-Wert von 1 bis 5 enthaltend in einem Träger eine Wirkstoffkombination aus
(a) mindestens einer organischen Verbindung der Formel (I)

   HS-X-COOM (I)

   worin
   - X: ein gesättigtes oder ungesättigtes, lineares oder verzweigtes sowie aliphatisches Kohlenwasserstoffgerüst bedeutet, das gegebenenfalls mit mindestens einer der folgenden Gruppen substituiert ist Thiolgruppe, Carboxylgruppe, Carboxylatgruppe, Hydroxygruppe, NH₂, (C₁ bis C₆)-Alkylamino, (C₁ bis C₆)-Dialkylamino, (C₁ bis C₆)-Hydroxyalkyl
   - M: steht für ein Wasserstoffatom, eine (C₁ bis C₈)-Alkylgruppe oder ein Äquivalent eines ein- oder mehrwertigen Kations, und
(b) mindestens einem cyclischen, organischen Carbonaten als organische Verbindung.

Unter einer derivatisierten Carboxylgruppe werden erfindungsgemäß bevorzugt Salze mit einem physiologisch verträglichen Kation, Carbonsäurester (-CO-O-R) und Carbonsäureamide (-CO-NH-R) verstanden. Hierbei steht R für einen gesättigten oder ungesättigten, linearen oder verzwelgten, cyclischen oder aromatischen, Kohlenwasserstoffrest, der gegebenenfalls substituiert sein kann.

Gemäß Formel (I) ist es bevorzugt, wenn X für Methylen, Ethan-1,1-diyl, Ethan-1,2-diyl, Propan-1,1-diyl, Propan-1,2-diyl, Propan-1,3-diyl oder Butan-1,4-diyl steht,
wobei jede dieser Gruppen gegebenenfalls mit mindestens einer der folgenden Reste substituiert sein kann Thiolgruppe, Carboxylgruppe, Carboxylatgruppe, Hydroxygruppe, -NH₂, (C₁ bis C₆)-Alkylaminogruppe, (C₁ bis C₆)-Dialkylaminogruppe.

Besonders bevorzugt steht in Formel (I) X für eine Gruppe aus
- Methylen,
- Ethan-1,1-diyl
- Ethan-1,2-diyl oder
- Propan-1,1-diyl steht,
   wobei jede dieser Gruppen gegebenenfalls mit mindestens einer der folgenden Reste substituiert sein kann Carboxylgruppe, Carboxylatgruppe, Hydroxygruppe, -NH2

Ganz besonders bevorzugt steht in Formel (I) X für eine Ethan-1,2-diyl-Gruppe wobei diese Gruppe mit mindestens einem der folgenden Reste substituiert ist Carboxylgruppe, Carboxylatgruppe, Hydroxygruppe, -NH₂

Jeweils gemäß Formel (I) zitierte (C₁ bis C₈)-Alkylreste (auch in den (C₁ bis C₆)-Alkylaminosowie (C₁ bis C₆)-Dialkylaminogruppen) stehen bevorzugt für (bzw. leiten sich bevorzugt ab von) Methyl, Ethyl, Propyl, Isopropyl, n-Butyl, sec-Butyl, iso-Butyl, tert.-Butyl, n-Pentyl, iso-Pentyl, sec-Pentyl, n-Hexyl, 2-Methylpentyl, n-Heptyl, n-Octyl, 6-Methylheptyl, 2-Ethylhexyl oder 1,1,3,3-Tetramethylbutyl.

Wenn die Verbindungen der Formel (I) als Salz vorliegen, steht M für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung vorliegenden Carboxylatfragments -COO⁻ in Formel (I). Das dafür zu verwendende Äquivalent des entsprechenden Kations beträgt 1/z. Das Fragment - COOM der Formeln (I) steht im Fall der Salzbildung für die Gruppe:

-COO⁻ 1/z (M^{z+})

Als ein- oder mehrwertiges Kation M^{z+} kommen prinzipiell alle physiologisch verträglichen Kationen in Frage. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht in Formel (I) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion. Besonders bevorzugt enthält das erfindungsgemäße Mittel als Komponente (a) mindestens eine Verbindung, ausgewählt aus mindestens einem Vertreter der Gruppe, die gebildet wird, aus L-Cystein (Säure oder Salz), D-Cystein (Säure oder Salz). D,L-Cystein (Säure oder Salz) und Acetylcystein. Ganz besonders bevorzugt geeignet ist jegliches Stereoisomer von Cystein jeweils als Säure oder Salz.

Die Komponente (a) ist in den erfindungsgemäßen Mitteln bevorzugt In einer Menge von 1 bis 10 Gew.-%, Insbesondere von 1 bis 5 Gew.-%, jeweils bezogen auf das Gewicht des gesamten Mittels, enthalten.

Als cyclisches, organisches Carbonat der Komponente (b) eignet sich erfindungsgemäß bevorzugt mindestens ein cyclischer Kohlensäureester. Diese cyclischen Ester der Kohlensäure leiten sich vom 1,3-Dioxolan-2-on ab und lassen sich durch folgende Grundstruktur der Formel (II-1) beschreiben; 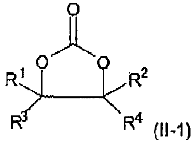 worin die Reste R¹, R², R³ und R⁴ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können.

Im Grundkörper, dem 1,3-Dioxolan-2-on, stehen die Reste R¹, R², R³ und R⁴ der Formel (II-1) jeweils für ein Wasserstoffatom. Weiterhin bevorzugt geeignete cyclische Kohlensäureester betreffen Derivate dieses Grundkörpers, wobei mindestens einer der Reste R¹, R², R³ und R⁴ der Formel (II-1) von einem Wasserstoffatom verschieden ist. Hierbei sind der strukturellen Vielfalt keine Grenzen gesetzt, so daß sich mono-, di-, tri- und tetra-substituierte 1,3-Dioxolan-2-one der Formel (II-1) zum Einsatz im Rahmen der vorliegenden Erfindung eignen.

Besonders bevorzugt sind neben dem unsubstituierten 1,3-Dioxolan-2-on insbesondere die in 4-Stellung monosubstituierten Derivate der nachstehenden Formel (II-2) 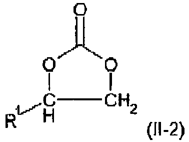 in der R¹ für einen substituierten oder unsubstituierten Alkyl-, Alkenyl- oder Alkylarylrest steht.

Bevorzugte Reste R¹ gemäß Formel (II-2) sind Methyl-, Ethyl-, n-Propyl-, iso-Propyl- sowie Hydroxymethyl-, 1-Nydroxyethyl- und 2-Hydroxyethyl-Reste.

Besonders bevorzugte erfindungsgemäße Mittel sind folglich dadurch gekennzeichnet, daß sie als 1,3-Dioxolan-2-on-Derivat mindestens eine Verbindung der obigen Formel (II-2) enthalten, bel der R¹ für einen substituierten oder unsubstituierten Alkyl-, Alkenyl- oder Alkylarylrest steht, wobei In weiter bevorzugten erfindungsgemäßen Mitteln der Rest R¹ in Formel (II-2) ausgewählt ist aus Methyl-, Ethyl-, n-Propyl-, iso-Propyl- sowie Hydroxymethyl-, 1-Hydroxyethyl- und 2-Hydroxyethyl-Resten.

Besonders bevorzugte 1,3-Dioxolan-2-one der Formel (II-1) stammen aus der Gruppe Ethylencarbonat (R¹, R², R³ und R⁴ = H), Propylencarbonat (R¹ = CH₃ sowie R², R³ und R⁴ = H) und Glycerincarbonat (R¹ = CH₂OH sowie R², R³ und R⁴ = H). Ganz besonders bevorzugt eignet sich Propylencarbonat.

Ethylencarbonat ist eine farblose kristalline Verbindung, die bei 39°C schmilzt und bei 238°C siedet. Das in Wasser, Alkoholen und organischen Lösungsmitteln leicht lösliche Ethylencarbonat ist über großtechnische Synthesen aus Ethylenoxid und flüssigem CO₂ herstellbar. Propylencarbonat ist eine wasserhelle, lelchtbewegliche Flüssigkeit, mit einer Dichte von 1,2057 gcm⁻³, der Schmelzpunkt liegt bei -49°C, der Siedepunkt bei 242°C. Auch Propylencarbonat Ist großtechnisch durch Reaktion von Propylenoxid und CO₂ bei 200°C und 80 bar zugänglich. Glycerincarbonat ist durch Umesterung von Ethylencarbonat oder Dimethylcarbonat mit Glycerin zugänglich, wobei als Nebenprodukte Ethylenglycol bzw. Methanol anfallen. Ein weiterer Syntheseweg geht von Glycidol (2,3-Epoxy-1-propanol) aus, das unter Druck in Gegenwart von Katalysatoren mit CO₂ zu Glycerincarbonat umgesetzt wird. Glycerincarbonat ist eine klare, leichtbewegliche Flüssigkeit mit einer Dichte von 1,398 gcm⁻³, die bei 125-130°C (0,15 mbar) siedet.

Die Verbindungen der Komponente (b) sind in dem erfindungsgemäßen Mittel bevorzugt in einer Menge von 5 Gew.-% bis 50 Gew.-%, insbesondere von 10 Gew.-% bis 30 Gew.%, jeweils bezogen auf das Gewicht des Mittels.

Die Gewichtsverhältnisse der Verbindungen der Formel (I) zu den Verbindungen der Komponente (b) betragen bevorzugt 1 zu 2 bis 1 zu 20, insbesondere 1 zu 3 bis 1 zu 10. besonders bevorzugt ist erfindungsgemäß mindestens einer der nachfolgenden Wirkstoffkombinationen in dem erfindungsgemäßen Mittel enthalten:

| | Komponente (a) | Komponente (b) |
|---|---|---|
| 1. | Cystein und/oder ein Salz davon | mindestens eine Verbindung der Formel (II-1) |
| 2. | Cystein und/oder ein Salz davon | mindestens eine Verbindung der Formel (II-2) |
| 3. | Cystein und/oder ein Salz davon | Ethylencarbonat |
| 3. | Cystein und/oder ein Salz davon | Glycerincarbonat |
| 4. | Cystein und/oder ein Salz davon | Propylencarbonat |

Eine weitere Verbesserung der Entfärbekraft stellt sich ein, wenn die erfindungsgemäßen Mittel neben der Wirkstoffkombination zusätzlich Oxalsäure enthalten. Dieser Zusatz erfolgt bevorzugt in einer Menge von 1 Gew.-% bis 5 Gew.-% bezogen auf das Gewicht des Mittels.

Dabei sind folgende Wirkstoffkombinationen bevorzugt:

| | Komponente (a) | Komponente (b) | |
|---|---|---|---|
| 1. | Cystein und/oder ein Salz davon | mindestens eine Verbindung der Formel (II-1) | Oxalsäure |
| 2. | Cystein und/oder ein Salz davon | mindestens eine Verbindung der Formel (II-2) | Oxalsäure |
| 3. | Cystein und/oder ein Salz davon | Ethylencarbonat | Oxalsäure |
| 3. | Cystein und/oder ein Salz davon | Glycerincarbonat | Oxalsäure |
| 4. | Cystein und/oder ein Salz davon | propylencarbonat | Oxalsäure |

Als Träger für ein anwendungsbereites Mitteln eignen sich bevorzugt flüssige Medien, wie beispielsweise Wasser oder organische Lösemittel, die verschieden von den Komponenten des erfindungsgemäßen Wirkstoffkomplexes sind. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines von den Verbindungen der Komponente (b) verschiedenen C₁-C₆-Alkohols, Insbesondere Ethanol bzw. Isopropanol, zu verstehen. Weitere alkoholische Lösemittel, sind beispielsweise Methoxybutanol, Benzylalkohol, 2-Phenoxyethenol, Methyldiglykol oder 1,2-Propylenglykol. In einer weiteren Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Das erfindungsgemäße Mittel besitzt bevorzugt einen pH-Wert von pH 1 bis pH 3.

Es ist ebenso erfindungsgemäß bevorzugt, wenn das erfindungsgemäße Mittel zusätzlich zur Effektsteigerung mindestens ein Redukton enthält. Unter einem Redukton versteht der Fachmann reduktiv wirkende Endiol-Verbindungen, die durch Substitution in α-Stellung stabilisiert sind und die der Tautomerie unterliegen. Die wichtigsten erfindungsgemäß einsetzbaren Reduktone sind Ascorbinsäure, Isoascorbinsäure, 2,3-Dihydroxy-2-propendial und 2,3-Dihydroxy-2-cyclopentenon.

Die Reduktone sind bevorzugt in einer Menge von 1,0 bis 5,0 Gew.-% bezogen auf das Gewicht des Mittels, in dem erfindungsgemäßen Mittel enthalten.

Dabei sind folgende Wirkstoffkombinationen bevorzugt:

| | Komponente (a) | Komponente (b) | Redukton |
|---|---|---|---|
| 1. | Cystein und/oder ein Salz davon | mindestens eine Verbindung der Formel (II-1) | Ascorbinsäure |
| 2. | Cystein und/oder ein Salz davon | mindestens eine verbindung der Formel (II-2) | Ascorbinsäure |
| 3. | Cystein und/oder ein Salz davon | Ethylencarbonat | Ascorbinsäure |
| 3. | Cystein und/oder ein Salz davon | Glycerincarbonat | Ascorbinsäure |
| 4. | Cystein und/oder ein Salz davon | Propylencarbonat | Ascorbinsäure |
| 5. | Cystein und/oder ein Salz davon | mindestens eine Verbindung der Formel (II-1) | Isoascorbinsäure |
| 6. | Cystein und/oder ein Salz davon | mindestens eine Verbindung der Formel (II-2) | Isoascorbinsäure |
| 7. | Cystein und/oder ein Salz davon | Ethylencarbonat | Isoascorbinsäure |
| 8. | Cystein und/oder ein Salz davon | Glycerincarbonat | Isoascorbinsäure |
| 9. | Cystein und/oder ein Salz davon | Propylencarbonat | Isoascorbinsäure |

Ferner steigert sich die Leistung der erfindungsgemäßen Mittel, wenn sie zusätzlich mindestens eine Oxocarbonsäure enthalten. Oxocarbonsäuren sind organische Verbindungen, die neben mindestens einer Carboxylgruppe eine Carbonylgruppe tragen und sind somit Aldehyd- bzw. Ketocarbonsäuren. Bevorzugte Oxocarbonsäuren sind α-Oxocarbonsäuren, ß-Oxocarbonsäuren, γ-Oxocarbonsäuren sowie ω-Oxocarbonsäuren. Darunter sind wiederum Verbindungen der Formel (V) bzw. deren Salze bevorzugt, 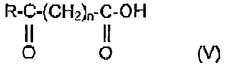 worin bedeuten
- R: ein Wasserstoffatom, eine (C₁ bis C₆)-Alkylgruppe, eine (C₁ bis C₆)-Hydroxyalkylgruppe, eine gegebenenfalls substituierte Arylgruppe, eine gegebenenfalls substituierte Heteroarylgruppe, eine (C₂ bis C₆)-Alkenylgruppe oder eine Carboxy-(C₁ bis C₆)-alkylgruppe,
- n: eine Zahl 0, 1, 2 oder 3.

Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren.

Dabei sind folgende Wirkstoffkombinationen bevorzugt:

| | Komponente (a) | Komponente (b) | Oxocarbonsäure |
|---|---|---|---|
| 1. | Cystein und/oder ein Salz davon | mindestens eine Verbindung der Formel (II-1) | Glyoxalsäure |
| 2. | Cystein und/oder ein Salz davon | mindestens eine Verbindung der Formel (II-2) | Glyoxalsäure |
| 3. | Cystein und/oder ein Salz davon | Ethylencarbonat | Glyoxalsäure |
| 3. | Cystein und/oder ein Salz davon | Glycerincarbonat | Glyoxalsäure |
| 4. | Cystein und/oder ein Salz davon | Propylencarbonat | Glyoxylsäure |
| 5. | Cystein und/oder ein Salz davon | mindestens eine Verbindung der Formel (II-1) | 3-Oxoglularsäure |
| 6. | Cystein und/oder ein Salz davon | mindestens eine Verbindung der Formel (II-2) | 3-Oxoglutarsäure |
| 7. | Cystein und/oder ein Salz davon | Ethylencarbonat | 3-Oxoglutarsäure |
| 8. | Cystein und/oder ein Salz davon | Glycerincarbonat | 3-Oxoglutarsäure |
| 9. | Cystein und/oder ein Salz davon | Propylencarbonat | 3-Oxoglutarsäure |

Die Oxocarbonsäuren sind bevorzugt in einer Menge von 1,0 bis 5,0 Gew.-% bezogen auf das Gewicht des Mittels, in dem erfindungsgemäßen Mittel enthalten.

Die in dem erfindungsgemäßen Verfahren eingesetzten kosmetischen Mittel können weiterhin alle für solche Zubereitungen bekannten Wirk-, Zusatz- und Hilfsstoffe enthalten:
Das Nachwaschen mit einem Shampoo entfällt, wenn ein stark tensidhaltiger Träger verwendet wurde.

In vieten Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, - phosphaten und/oder -isethionaten, die sich von Alkyl- und/oder Alkenyloligoglykosiden der allgemeinen Formel (VI) ableiten,

   R-O-(G)ₚ (VI)

   mit der Bedeutung
   - R: C₆₋₂₂-Alkyl oder C₆₋₂₂-Alkenyl,
   - G: Glykosideinheit, die sich von einem Zucker mit 5 oder 6 Kohlenstoffatomen ableitet,
   - p: Zahl von 1 bis 10,
   insbesondere das Laurylglucosidcarboxylat, wie es als Plantapon^{®} LGC von Cognis Deutschland erhältlich ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)x. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹ im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾- Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine-COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCl-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Die erfindungsgemäßen Mittel können zur Verstärkung der Faserpflege ohne Reduzierung der Entfärbekraft zusätzlich mindestens ein Silikon enthalten. Die Silikone, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone ausgewählt, aus mindestens einem Vertreter aus der Liste, die gebildet wird aus:
(i)Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter: substituierten oder unsubstituierten aminierten Gruppen; (per)fluorierten Gruppen; Thiolgruppen; Carboxylatgruppen; hydroxylierten Gruppen; alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCl-Bezeichnung Bis-PEG/PPG-20/20 Dimethicone vertriebene Handelsprodukt Abil B 8832 der Firma Degussa;
(vi) oder deren Gemischen.

Besonders bevorzugte erfindungsgemäße kosmetische oder dermatologische Zubereitungen sind dadurch gekennzeichnet, dass sie mindestens ein Silikon der Formel (Si-1)

(CH₃)₃Si-[O-Si(CH₃)₂]ₓ-O-Si(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten kosmetischen oder dermatologischen Zubereitungen enthalten ein Silikon der vorstehenden Formel (Si-1). Diese Silikone werden nach der INCI-Nomenklatur als Dimethicone bezeichnet.

Selbstverständlich können auch Mischungen der o.g. Silikone in den bevorzugten erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})ₓ(R_{c}SiO_{(4-c)}/₂)_{y}M (Si-2)

beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist, worin
R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
Z ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-,-OCH₂ CH₂CH₂-, -CH₂CH(CH₃)C(O)OCH₂-, -(-CH₂)₃ CC(O)OCH₂CH₂-, -C₆H ₄C₆H₄-, -C₆H ₄CH₂C₆H₄-; und -(CH₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist - NH(CH₂)_{z}(CH₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist -N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel -CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO _{(4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte erfindungsgemäße kosmetische oder dermatologische Zubereitungen enthalten ein aminofunktionelles Silikon der Formel (Si-3)

R'ₐG₃₋ₐ-Si(OSiG₂)ₙ-(OSiG _{b}R'_{2-b})ₘ-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)_{3 ;}
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus -Q-N(R")-CH₂-CH₂-N(R")₂, -Q-N(R")₂, -Q-N⁺(R")₃A⁻ , -Q-N⁺H(R")₂ A⁻, -Q-N⁺H₂(R")A⁻ , -Q-N(R")-CH₂-CH₂-N⁺R"H₂A⁻, wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und Aein Anion repräsentiert, welches vorzugsweise ausgewählt ist aus Chlorid, Bromid, lodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCl-Bezeichnung: Aminopropyl Phenyl Trimethicone), DC 5-7113 (Hersteller Dow Corning, INCl-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Besonders bevorzugte erfindungsgemäße Mittel sind dadurch gekennzeichnet, dass sie mindestens es ein aminofunktionelles Silikon der Formel (Si3-a) 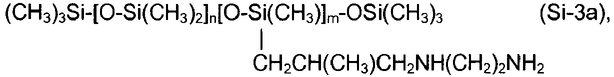 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCl-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 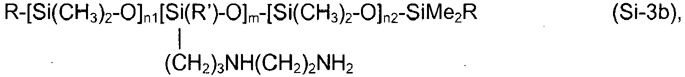 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCl-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silikone eingesetzt werden, sind erfindungsgemäße kosmetische oder dermatologische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCl als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 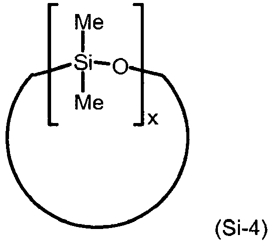 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfasst dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Sihaltige Gruppen, O-haltige Gruppen usw.. Vorzugsweise sind mindestens 80% der Reste R Alkylreste, besonders bevorzugt Methylgruppen.

Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel der Ausführungsform mit einem Silikon sind dadurch gekennzeichnet, dass das Silikon wasserlöslich ist.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 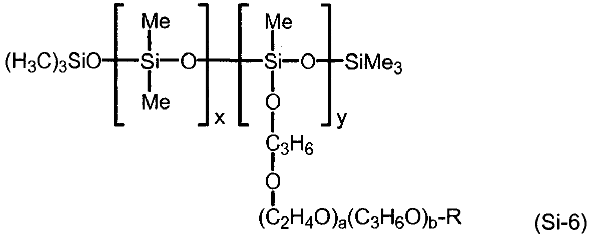 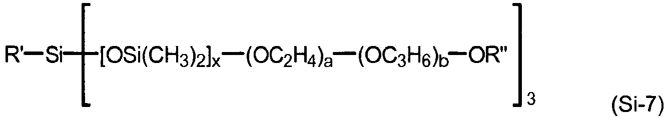 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193 (Dow).

Desweiteren enthält das erfindungsgemäße Mittel bevorzugt zur Haarkonditionierung mindestens ein kationisches Polymer. Erfindungsgemäße Mittel, die ein solches Polymer enthalten, erleiden keinen Leistungsabfall der Entfärbekraft, sondern erfahren sogar eine leichte Wirkungssteigerung.

Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 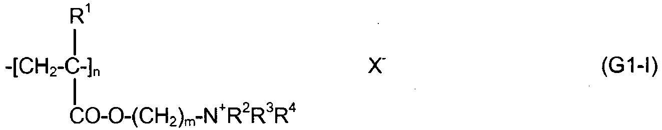 in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCl-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCl-Bezeichnung: Mineral Oil) und Tridecyl-polyoxypropylen-polyoxyethylen-ether (INCl-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCl-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (M-I),

   R¹-CH=CR²-CO-Z-(CₙH₂ₙ)-N⁽⁺⁾R³R⁴R⁵ A⁽⁻⁾ (M-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (M-II),

   R⁶-CH-CR⁷-COOH (M-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen farbverändernden Mittel enthalten die kationischen Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Das erfindungsgemäße Mittel ist bevorzugt besonders wirksam, wenn es eine Viskosität von 500 bis 30000 mPa·s, insbesondere von 1000 bis 10000 mPa·s aufweist (jeweils Brookfield DV-II+, Spindel 4, 20 UpM bei 20°C).

Dem erfindungsgemäßen Mittel wird somit bevorzugt mindestens ein Verdickungsmittel zugesetzt. Zur Verdickung der erfindungsgemäßen Mittel eignen sich bevorzugt verdickende Polymere. In wäßrigen Phasen beruht ihre die Viskosität erhöhende Funktion auf ihrer Löslichkeit in Wasser oder ihrer hydrophilen Natur. Die erfindungsgemäß zur Verdickung eingesetztem Polymere werden sowohl in tensidischen als auch in emulsionsförmigen Systemen angewendet.

Bevorzugt finden polymere Verdicker Verwendung, die bei einem sauren pH-Wert eine verdickende Wirkung, bevorzugt im angegebenen bevorzugten Viskositätsbereich, ausüben.

Besonders bevorzugt geeignete verdickende Polymere werden ausgewählt aus Xanthangum und oder Cellulose bzw. Derivaten der Cellulose, insbesondere Cellulosether.

Zur Einstellung der Viskosität enthalten die erfindungsgemäßen Mittel ganz besonders bevorzugt mindestens ein verdickendes Polymer ausgewählt unter Hydroxyethylcellulose (z.B. Natrosol^{®} 250 HR der Fa. Hercules), Hydroxypropylcellulose (z.B. Klucel PR^{®} der Fa. Hercules), Methylhydroxyethylcellulose (z.B. Culminal^{®} MHEC 8000 der Fa. Hercules), Methylhydroxypropylcellulose (z.B. Benecel^{®} MP 943 R der Fa. Hercules) und Hexadecylhydroxyethylcellulose (z.B. Natrosol^{®} plus grade 330 PA der Fa. Hercules).

Die zu entfärbenden keratinhaltigen Fasern sind bevorzugt mit Oxidationsfarbstoffen und/oder direktziehenden Farbstoffen, als Vertreter der synthetischen Farbstoffe, gefärbt.

Als zur Färbung der zu entfärbenden keratinhaltigen Fasern verwendeten Entwicklerkomponenten können prinzipiell nachfolgende Entwicklerkomponenten dienen.

Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin zur Färbung des Substrats bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze zur Färbung der zu entfärbenden keratinhaltigen Fasern einzusetzen.

Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente zur Färbung der zu entfärbenden keratinhaltigen Fasern ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methyl-pyrazol, 4,5-Diamino-3-hydroxymethyl-1-isopropylpyrazol, 4,5-Diamino-3-methyl-1-isopropyl-pyrazol, 4-Amino-5-(β-aminoethyl)amino-1,3-dimethylpyrazol, sowie deren physiologisch verträglichen Salze.

Unter den Pyrazolo[1,5-a]pyrimidinen der obenstehenden Formel (E7) kann man insbesondere nennen: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]pyrimidin-7-ylamino)-ethanol; 2-(7-Aminopyrazolo[1,5-a]pyrimidin-3-ylamino)-ethanol; 2-[(3-Aminopyrazolo[1,5-a]pyrimidin-7-yl)-(2-hydroxy-ethyl)-amino]-ethanol; 2-[(7-Aminopyrazolo[1,5-a]pyrimidin-3-yl)-(2-hydroxy-ethyl)-amino]-ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin; sowie ihre physiologisch verträglichen Salze und ihre tautomeren Formen, wenn ein tautomers Gleichgewicht vorhanden ist.

Ganz besonders bevorzugte Entwicklerkomponenten zur Färbung der zu entfärbenden keratinhaltigen Fasern werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraamino-pyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass zur Färbung der zu entfärbenden keratinhaltigen Fasern bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten zur Färbung der zu entfärbenden keratinhaltigen Fasern werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Zur Färbung der zu entfärbenden keratinhaltigen Fasern werden erfindungsgemäß besonders bevorzugte Kupplerkomponenten ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das zur Färbung der zu entfärbenden keratinhaltigen Fasern angewendete Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Weiterhin können die zu entfärbenden keratinhaltigen Fasern auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Die zu entfärbenden keratinhaltigen Fasern können entweder mit direktziehenden Farbstoffe allein oder in Kombination von direktziehenden Farbstoffen mit Oxidationsfarbstoffen gefärbt worden sein. Bevorzugt geeignete direktziehende Farbstoffe sind Nitrophenylendiamine, Nitroaminophenole, Azofarbstoffe, Anthrachinone oder Indophenole. Bevorzugt zur Entfärbung geeignete direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugte erfindungsgemäße Mittel sind dadurch gekennzeichnet, daß sie zusätzlich mindestens einen direktziehenden Farbstoff, vorzugsweise in einer Menge von 0,01 bis 20 Gew.-%, bezogen auf das gesamte Färbemittel, enthalten.

Ferner können die erfindungsgemäß zu entfärbenden keratinhaltigen Fasern bevorzugt mit einem kationischen direktziehenden Farbstoff gefärbt worden sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ein zweiter Gegenstand der Erfindung ist ein Verfahren zur reduktiven Entfärbung von keratinhaltigen Fasern, insbesondere menschlichen Haaren, in dem ein Mittel des ersten Erfindungsgegenstandes auf die keratinhaltigen Fasern aufgetragen wird und nach einer Einwirkzeit wieder abgespült wird.

Die keratinhaltigen Fasern wurden bevorzugt mit Oxidationsfarbstoffen und/oder direktziehenden Farbstoffen eingefärbt.

Die Einwirkzeit beträgt bevorzugt 1 bis 60 Minuten, bevorzugt 5 bis 30 Minuten. Die Einwirkung des erfindungsgemaßen Mittels kann nicht nur bei Raumtemperatur, sondern bevorzugt in einem Temperaturbereich von 15 bis 60 °C, Insbesondere von 25 bis 60 °C erfolgen.

In einer bevorzugten Ausführungsform des Verfahrens wird unmittelbar vor dem Auftragen des erfindungsgemäßen Mittels des ersten Erfindungsgegenstandes das anwendungsbereite Mittel durch mischen einer Zusammensetzung, enthaltend, gegebenenfalls in einem kosmetischen Träger, mindestens eine organische Verbindung der Formel (I) (vide supra) mit einer Zusammensetzung, enthaltend In einem kosmetischen Träger, mindestens eine organische Verbindung der cyclischen, organischen Carbonate hergestellt.

Dabei können besagte Zusammensetzungen vor der Vermischung wie weiter unten beschrieben konfektioniert bzw. beschaffen sein.

Nach Ablauf der Einwirkzeit werden die keratinhaltigen Fasern ausgespült, wobei bevorzugt ein tensldhaltiges Mittel, wie beispielswelse ein Reinigungsmittel oder ein Shampoo, Anwendung findet. Gegebenenfalls kann das Substrat mehrfach ausgespült, bzw. mit dem tensidhaltigen Mittel behandelt werden.

Nach dem Ausspülen kann es vorteilhaft sein, die keratinhaltigen Fasern mit einer Oxidationsmittelhaltigen Zusammensetzung zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Es ist erfindungsgemäß bevorzugt das erfindungsgemäße Mittel als Mehrkomponontensystem in Form eines Kit-of-parts bereitzustellen. Dabei Ist es bevorzugt, dass in einem ersten Kontainer eine Zusammensetzung, enthaltend, gegebenenfalls in einem kosmetischen Träger, mindestens eine organische Verbindung der Formel (I) (vide supra) und in einem davon getrennten zweiten Kontainer eine Zusammensetzung, enthaltend in einem kosmetischen Träger, mindestens eine organische Verbindung der cyclischen, organischen Carbonate konfektioniert ist.

Als Kontainer verstehen sich erfindungsgemäß Behältnisse wie beispielswelse Flaschen, Sachets, Beutel, Tuben, Dosen und viele andere mehr. Auch eine Kammer eines Mehrkammerbehältnisses gilt im Sinne der Erfindung als Kontainer. In einem Mehrkammerbehältnis werden die jeweiligen Zusammensetzungen in unterschiedlichen Kammern konfektioniert und worden unmittelbar vor dem Austritt aus dem Mehrkammerbehältnis oder danach zusammengebracht und dadurch gemischt.

Die Zusammensetzung des ersten Kontainers liegt bevorzugt als Festkörper, insbesondere pulverförmig, granuliert oder als Formkörper, vor.

Eine pulverförmige Zusammensetzung des ersten Kontainers besitzt eine bevorzugte mittlere Teilchengröße von 0,0001 bis 100 µm, insbesondere von 0,005 bis 10 µm. Diese Pulver können durch Beschichtung mit Fetten, Ölen oder Wachsen, wie beispielswelse Silikonölen, flüssigen Kohlenwasserstoffen, Dialkylethern, Fettsäuren, Fettalkoholen, entstaubt werden.

Als Granulate werden erfindungsgemäß körnige Partikel verstanden. Diese körnigen Partikel sind fließfähig.

Granulate können durch Feuchtgranulierung, durch Trockengranulierung bzw. Kompaktierung und durch Schmelzerstarrungsgranullerung hergestellt werden. Die gebräuchlichste Granuliertechnik ist die Feuchtgranulierung, da diese Technik den wenigsten Einschränkungen unterworfen ist und am sichersten zu Granulaten mit günstigen Eigenschaften führt. Die Feuchtgranulierung erfolgt durch Befeuchtung der Pulvermischungen mit Lösungsmitteln und/oder Lösungsmittelgemischen und/oder Lösungen von Bindemitteln und/oder Lösungen von Klebstoffen und wird vorzugsweise in Mischern, Wirbelbetten oder Sprühtürmen durchgeführt, wobei besagte Mischer beispielsweise mit Rühr- und Knetwerkzeugen ausgestattet sein können. Für die Granulation sind jedoch auch Kombinationen von Wirbelbett(en) und Mischer(n), bzw. Kombinationen verschiedener Mischer einsetzbar. Die Granulation erfolgt unter Einwirkung niedriger bis hoher Scherkräfte.

Wenn Die Zusammensetzung des ersten Kontainers als Formkörper vorliegt, dann können diese erfindungsgemäßen Formkörper jedwede geometrische Form aufweisen, Besonders bevorzugt sind Formkörper In Gestalt sphärischer Geometrie.

In einer weiteren bevorzugten Ausführungsform werden daher nahezu kugelförmige Formkörper, insbesondere durch Extrusion und nachfolgender Verrundung zur Formgebung, hergestellt.

Ein erfindungsgemäßer Formkörper besitzt eine bevorzugte Bruchhärte von 30 - 100 N, besonders bevorzugt von 40 - 80 N, ganz besonders bevorzugt von 50 - 60 N (gemessen nach Europäisches Arzneibuch 1997, 3. Ausgabe, ISBN 3-7692-2186-9, "2.9.8 Bruchfestigkeit von Tabletten"; Seite 143-144 mit einem Tablettenhärte-Prüfgerät Schleuniger 6D).

Unabhängig von seiner Erscheinungsform als Pulver, Granulat bzw. Formkörper enthält Die Zusammensetzung des ersten Kontainers bevorzugt mindestens einen der nachfolgenden Zusätze:
Die Zusammensetzung des ersten Kontainers enthält bevorzugt zusätzlich mindestens einen Auflösungsbeschleuniger. Dies ist insbesondere dann bevorzugt, wenn Die Zusammensetzung des ersten Kontainers granuliert oder als Formkörper vorliegt. Der Begriff Auflösungsbeschleuniger umfasst dabei Gas-entwickelnde Komponenten, vorgebildete und eingeschlossene Gase, Sprengmittel sowie deren Mischungen.

In einer ersten Ausführungsform der vorliegenden Erfindung werden als Auflösungsbeschleuniger Gas-entwickelnde Komponenten eingesetzt. Diese Komponenten reagieren bei Kontakt mit Wasser miteinander unter in-situ Bildung von Gasen, die in der Tablette einen Druck erzeugen, der die Tablette in kleinere Partikel zerfallen läßt. Ein Beispiel für ein derartiges System sind spezielle Kombinationen von geeigneten Säuren mit Basen. Bevorzugt sind ein-, zwei- oder dreiwertige Säuren mit einem pKₐ-Wert von 1,0 bis 6,9. Bevorzugte Säuren sind Citronensäure, Äpfelsäure, Maleinsäure, Malonsäure, Itaconsäure, Weinsäure, Oxalsäure, Glutarsäure, Glutaminsäure, Milchsäure, Fumarsäure, Glykolsäure sowie deren Mischungen. Besonders bevorzugt ist Citronensäure. Ganz besonders bevorzugt kann es sein, die Citronensäure in Teilchenform einzusetzen, wobei die Teilchen einen Durchmesser unterhalb von 1000µm, insbesondere kleiner als 700µm, ganz besonders bevorzugt kleiner als 400µm, aufweisen. Weitere alternative geeignete Säuren sind die Homopolymere oder Copolymere von Acrylsäure, Maleinsäure, Methacrylsäure oder Itaconsäure mit einem Molekulargewicht von 2000 bis 200 000. Besonders bevorzugt sind Homopolymere der Acrylsäure und Copolymere aus Acrylsäure und Maleinsäure. Bevorzugte Basen sind erfindungsgemäß Alkalimetallsilikate, Carbonate, Hydrogencarbonate sowie deren Mischungen. Metasilicate, Hydrogencarbonate und Carbonate sind besonders bevorzugt, Hydrogencarbonate sind ganz besonders bevorzugt. Besonders bevorzugt sind teilchenförmige Hydrogencarbonate mit einem Teilchendurchmesser von weniger als 1000µm, insbesondere weniger als 700µm, ganz besonders bevorzugt weniger als 400µm. Natrium oder Kaliumsalze der oben genannten Basen sind besonders bevorzugt. Diese Gasentwickelnden Komponenten sind in den erfindungsgemäßen Färbeformkörpern bevorzugt in einer Menge von mindestens 10 Gew.-%, insbesondere von mindestens 20 Gew.-%, enthalten.

In einer weiteren Ausführungsform der vorliegenden Erfindung ist das Gas vorgebildet oder eingeschlossen, so daß bei Einsetzen der Auflösung des Formkörpers die Gasentwicklung beginnt und die weitere Auflösung beschleunigt. Beispiele geeigneter Gase sind Luft, Kohlendioxid, N₂O, Sauerstoff und/oder weitere nicht-toxische, nicht-brennbare Gase.

In einer besonders bevorzugten Ausführungsform der vorliegenden Erfindung werden als Auflösungsbeschleuniger Desintegrationshilfsmittel, sogenannte Sprengmittel, in die Zusammensetzung des ersten Kontainers eingearbeitet, um die Zerfallszeiten zu verkürzen. Dies ist insbesondere dann bevorzugt, wenn die Zusammensetzung des ersten Kontainers als Formkörper oder als Granulat vorliegen. Unter Sprengmitteln bzw. Zerfallsbeschleunigern werden gemäß Römpp (9. Auflage, Bd. 6, S. 4440) und Voigt "Lehrbuch der pharmazeutischen Technologie" (6. Auflage, 1987, S. 182-184) Hilfsstoffe verstanden, die für den raschen Zerfall von Feststoffagglomeraten - das heißt insbesondere von Formkörpern - in Wasser oder Magensaft und für die Freisetzung der Pharmaka in resorbierbarer Form sorgen.

Diese Stoffe, die auch aufgrund ihrer Wirkung als "Spreng" mittel bezeichnet werden, vergrößern bei Wasserzutritt ihr Volumen (Quellung). Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden im Rahmen der vorliegenden Erfindung Desintegrationsmittel auf Cellulosebasis eingesetzt, so daß bevorzugte Zusammensetzungen des ersten Kontainers - insbesondere wenn es als Formkörper vorliegt - in solches Desintegrationsmittel auf Cellulosebasis in Mengen von 0,5 bis 70 Gew.-%, vorzugsweise 3 bis 30 Gew.-%, bezogen auf die gesamte Zusammensetzung des ersten Kontainers enthalten. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose kann erfindungsgemäß nicht in feinteiliger Form eingesetzt, sondern vor dem Zumischen zu den zu verpressenden Vorgemischen in eine gröbere Form überführt, beispielsweise granuliert oder kompaktiert. Die Teilchengrößen solcher Desintegrationsmittel liegen zumeist oberhalb 200 µm, vorzugsweise zu mindestens 90 Gew.-% zwischen 300 und 1600 µm und insbesondere zu mindestens 90 Gew.-% zwischen 400 und 1200 µm. Die erfindungsgemäßen Desintegrationshilfsmittel sind beispielsweise im Handel unter der Bezeichnung Arbocel^{®} von der Firma Rettenmaier erhältlich. Ein bevorzugtes Desintegrationshilfsmittel ist beispielsweise Arbocel^{®}TF-30-HG.

Als bevorzugtes Desintegrationsmittel auf Cellulosebasis oder als Bestandteil dieser Komponente wird mikrokristalline Cellulose verwendet. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Geeignete mikrokristalline Cellulose ist beispielsweise unter dem Handelsnamen Avicel^{®} kommerziell erhältlich.

Die beschleunigte Auflösung der Zusammensetzung des ersten Kontainers kann erfindungsgemäß auch durch Vorgranulierung der weiteren Bestandteile erreicht werden.

In einer bevorzugten Ausführungsform der erfindungsgemäßen Zusammensetzungen des ersten Kontainers enthalten diese zur Auflösungsbeschleunigung, insbesondere neben mindestens einem Sprengmittel auf Cellulosebasis, ein Gemisch aus Stärke und mindestens einem Saccharid. Disaccharide sind bevorzugt verwendete Saccharide dieser Ausführungsform. Das besagte Gemisch liegt bevorzugt in einem Gewichtsverhältnis von Stärke und den eingesetzten Sacchariden von 10 : 1 bis 1 : 10, besonders bevorzugt von 1 : 1 bis 1 : 10, ganz besonders bevorzugt von 1 : 4 bis 1 : 8 in der Zusammensetzung des ersten Kontainers vor.

Die verwendeten Disaccharide sind bevorzugt ausgewählt aus Lactose, Maltose, Saccharose, Trehalose, Turanose, Gentiobiose, Melibiose und Cellobiose. Besonders bevorzugt werden Lactose, Maltose und Saccharose und ganz besonders bevorzugt Lactose in den erfindungsgemäßen Formkörpern eingesetzt.

Die Stärke-Saccharid-Mischung ist in der Zusammensetzung des ersten Kontainers in einer Menge von 5 bis 70 Gew.%, bevorzugt von 20 bis 40 Gew.% bezogen auf die Masse des gesamten Mittels A, enthalten.

Die Zusammensetzung des zweiten Kontainers umfasst bevorzugt einem bei Anwendungsbedingungen flüssigen kosmetischen Träger. Dies gilt insbesondere dann, wenn die Zusammensetzung des ersten Kontainers pulverförmig, granuliert oder als Formkörper vorliegt.

Das Kit kann zusätzlich Applikationshilfen, wie beispielsweise Pinsel oder Mascarabürste, enthalten.

Das Kit kann zusätzlich Schutzhandschuhe enthalten.

Das Kit kann weiterhin zusätzlich einen Konditioner und/oder ein Shampoo enthalten.

Für die Erfindungsgegenstände zwei bis drei gelten *mutatis mutandis* die Definitionen und Ausführungsformen, die im Rahmen des ersten Erfindungsgegenstandes Erwähnung finden.

Der Erfindungsgegenstand soll exemplarisch anhand der folgenden Ausführungen erläutert werden.

### Beispiele

### 1. Herstellung der Rezepturen

Der Rohstoff Natrosol 250^{®} HHR wurde in Wasser vorgequollen. Die Rohstoffe Brij^{®} 30 und Eumulgin^{®} L, bzw. Cremophor^{®} CO 40, Eumulgin^{®} L und Euxyl^{®} PE 9010, wurden vermischt und anschließend das Propylencarbonat untergerührt. Diese Mischung wurde sodann unter Rühren zu der Natrosolquellung gegeben. Unmittelbar vor der Anwendung am gefärbten Haar wurde die Mischung aus L-Cysteinhydrochloridmonohydrat und Oxalsäure zugefügt.

Die Mengenangaben sind Gewichtsprozent bezogen auf das Gewicht des jeweiligen Mittels.

**Tabelle 1: Rezepturen**

| Rohstoff | E1 | E2 | E3 | V1 | V2 |
|---|---|---|---|---|---|
| L-Cysteinhydrochlorinmonohydrat | 3,00 | 3,00 | 3,00 | 3,00 | - |
| Propylencarbonat | 20,00 | 20,00 | 20,00 | - | 20,00 |
| Oxalsäure | 1,00 | 1,00 | - | 1,00 | 1,00 |
| Ascorbinsäure | - | - | 1,00 | - | - |
| Brij^{®} 30 ¹ | 2,00 | - | 2,00 | 2,00 | 2,00 |
| Cremophor^{®} CO 40 ² | - | 2,00 | - | - | - |
| Euxyl^{®} PE 9010 ³ | - | 1,00 | - | - | - |
| Eumulgin^{®} L ⁴ | 2,00 | 2,00 | 2,00 | 2,00 | 2,00 |
| Natrosol^{®} 250 UHR ⁵ | 1,25 | 1,25 | 1,25 | 1,25 | 1,25 |
| Wasser | ad 100 | ad 100 | ad 100 | ad 100 | ad 100 |

| | | | | | |
|---|---|---|---|---|---|
| ¹ Mit 4 Einheiten Ethylenoxid ethoxylierter Dodecylalkohol (100 Gew.-% Aktivsubstanz; INCI-Bezeichnung: Laureth-4) (Unichema) ² hydriertes Rizinusöl mit ca. 40-45 EO-Einheiten (100 Gew.-% Aktivsubstanz; INCI-Bezeichnung: PEG-40 Hydrogenated Castor Oil) (BASF) ³ Mischung aus 90 Gew.-% 2-Phenoxyethanol und 10 Gew.-% 3-(2-Ethylhexyloxy)-1,2-propandiol (100 Gew.-% Aktivsubstanz, INCI-Bezeichung: Phenoxyethanol, Ethylhexyl Glycerin) (Schülke & Mayr) ⁴ Laurylglykolether, ethoxyliert mit 1 Einheit Propylenoxid und 9 Einheiten Ethylenoxid (INCI-Bezeichnung: PPG-1-PEG-9 Lauryl Glycol Ether) (Cognis) ⁵ Hydroxyethylcellulose (INCI-Bezeichnung: Hydroxyethylcellulose) (Hercules) 2.0 Entfärbetests | | | | | |

2 Gewichtsteile des jeweiligen in Tabelle 2 gelisteten Färbemittels der Marke Igora Royal (Schwarzkopf) wurde bei Raumtemperatur und 30 min Einwirkzeit jeweils auf 1 Gewichtsteil Büffelbauchhaar (Fa. Hohenschildt, Berlin) ausgefärbt. Anschließend wurde das Haar gespült und getrocknet. Danach wurden die Strähnen farbmetrisch mit dem Messgerät Datacolor SF 6000X (Fa. Datacolor) vermessen und deren Farbstärke als Referenzwert (gefärbte Strähne = Farbstärke entspricht 100 %) bestimmt.

Je Färbemittel wurden 12 Haarsträhnen ausgefärbt. Alle Werte für die Farbstärke vor und nach dem Entfärben wurden gemittelt (arithmetisches Mittel).

Anschließend wurden pro Entfärbemittel E1, V1 und V2 jeweils 4 der gefärbten Haarsträhnen unter jeweils gleichen Bedingungen entfärbt. Die Strähnen wurden mit Wasser gespült und getrocknet. Abschließend wurde wiederum farbmetrisch die Restfarbstärke nach dem Entfärben bestimmt. die Restfarbstärken wurden in Tabelle 2 zusammengefasst.

**Tabelle 2: Restfarbstärken nach dem Entfärben in % zur Farbstärke der Ausgangsfärbung**

| Färbemittel | E1 | V1 | V2 |
|---|---|---|---|
| Igora Royal 6-0 | 48,22 | 59,72 | 70,82 |
| Igora Royal 6-7 | 28,38 | 39,92 | 75,82 |
| Igora Royal 6-888 | 46,33 | 57,04 | 59,75 |
| Igora Royal 4-90 | 40,58 | 80,69 | 84,16 |
| Igora Royal 0-77 | 22,04 | 51,08 | 50,45 |

Es ist deutlich erkennbar, dass die Restfarbstärken der mit dem erfindungsgemäßen Entfärbemittel behandelten Haarsträhnen im Vergleich zu den Mitteln des Standes der Technik deutlich geringer ausfallen. Die Entfärbekraft der erfindungsgemäßen Entfärbemittel ist folglich größer.

Die Entfärbung mit den Mitteln E2 und E3 sind zu denen des Mittels E1 vergleichbar.

## Patentansprüche

1. Verwendung eines Mittels mit einem pH-Wert von 1 bis 5, enthaltend in einem Träger eine Wirkstoffkombination aus
(a) mindestens einer organischen Verbindung der Formel (I)
HS-X-COOM (I)
worin
X ein gesättigtes oder ungesättigtes, lineares oder verzweigtes sowie aliphatisches Kohlenwasserstoffgerüst bedeutet, das gegebenenfalls mit mindestens einer der folgenden Gruppen substituiert ist Thiolgruppe, Carboxylgruppe, Carboxylatgruppe, Hydroxygruppe, -NH₂, (C₁ bis C₆)-Alkylamino, (C₁ bis C₆)-Dialkylamino, (C₁ bis C₆)-Hydroxyalkyl,
M steht für ein Wasserstoffatom, eine (C₁ bis C₈)-Alkylgruppe oder ein Äquivalent eines ein- oder mehrwertigen Kations,
und
(b) mindestens einem cyclischen, organischen Carbonat als organische Verbindung, zur reduktiven Entfärbung keratinhaltiger Fasern, insbesondere menschlicher Haare.

2. Verwendung nach Anspruch 1, **dadurch gekennzeichnet, daß** Komponente (a) ausgewählt wird aus mindestens einem Vertreter der Gruppe, die gebildet wird, aus L-Cystein (Säure oder Salz), D-Cystein (Säure oder Salz), D,L-Cystein (Säure oder Salz) und Acetylcystein

3. Verwendung nach einem der Ansprüche 1 oder 2, **dadurch gekennzeichnet, daß** die Komponente (b) ausgewählt wird, aus mindestens einem cyclischer Kohlensäureester der Formel (II-1) 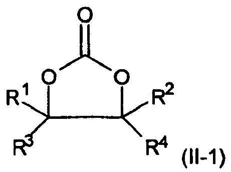 worin die Reste R¹, R², R³ und R⁴ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können

4. Verwendung nach einem der Ansprüche 1 bis 3, **dadurch gekennzeichnet, daß** das Mittel die Verbindungen der Komponente (a) in einer Menge von 1 bis 10 Gew.-%, insbesondere von 1 bis 5 Gew.-%, jeweils bezogen auf das Gewicht des gesamten Mittels, enthält.

5. Verwendung nach einem der Ansprüche 1 bis 6, **dadurch gekennzeichnet, daß** das Mittel die Verbindungen der Komponente (b) in einer Menge von 5 Gew.-% bis 50 Gew.-%, insbesondere von 10 Gew.-% bis 30 Gew.-%, jeweils bezogen auf das Gewicht des Mittels, enthält.

6. Verwendung nach einem der Ansprüche 1 bis 5, **dadurch gekennzeichnet, daß** das Mittel einen pH-Wert von pH 1 bis pH 3, besitzt.

7. Verwendung nach einem der Ansprüche 1 bis 6, **dadurch gekennzeichnet, daß** das Mittel zusätzlich Oxalsäure enthält.

8. Verwendung nach einem der Ansprüche 1 bis 7, **dadurch gekennzeichnet, daß** das Mittel zusätzlich mindestens ein Redukton enthält.

9. Verwendung nach einem der Ansprüche 1 bis 8 **dadurch gekennzeichnet, daß** das Mittel mindestens ein verdickendes Polymer enthält.

10. Verfahren zur reduktiven Entfärbung von keratinhaltigen Fasern, insbesondere menschlichen Haaren, in dem ein in einem der Ansprüche 1 bis 9 beschriebenes Mittel auf die keratinhaltigen Fasern aufgetragen wird und nach einer Einwirkzeit wieder abgespült wird.

## Claims

1. A use of an agent having a pH of 1 to 5, containing, in a vehicle, an active ingredient combination of
(a) at least one organic compound of formula (I)
**HS-X-COOM** **(I)**
wherein
X denotes a saturated or unsaturated, linear or branched and aliphatic hydrocarbon structure, which is optionally substituted with at least one of the following groups: thiol group, carboxyl group, carboxylate group, hydroxyl group, -NH₂, (C₁ to C₆)-alkylamino, (C₁ to C₆)-dialkylamino, (C₁ to C₆)-hydroxyalkyl,
M stands for a hydrogen atom, a (C₁ to C₈)-alkyl group or an equivalent of a monovalent or polyvalent cation,
and
(b) at least cyclic organic carbonate as the organic compound, for reductive decolouration of keratin-containing fibers, in particular human hair.

2. The use according to claim 1, **characterized in that** component (a) is selected from at least one representative of the group formed by L-cysteine (acid or salt), D-cysteine (acid or salt), D,L-cysteine (acid or salt) and acetylcysteine.

3. The use according to one of claims 1 or 2, **characterized in that** component (b) is selected from at least one cyclic carbonic acid ester of formula (II-1) 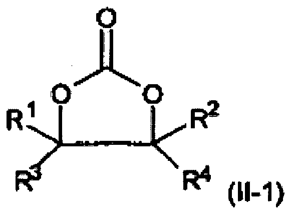 wherein the residues R¹, R², R³ and R⁴, independently of one another, stand for a hydrogen atom or organic residues, in particular alkyl, alkenyl or alkylaryl, which may additionally be substituted with other groups, in particular hydroxyl groups.

4. The use according to one of claims 1 to 3, **characterized in that** the agent contains the compounds of component (a) in an amount of 1% to 10% by weight, in particular 1% to 5% by weight, each based on the weight of the total agent.

5. The use according to one of claims 1 to 6 [sic; 4?], **characterized in that** the agent contains the compounds of component (b) in an amount of 5% by weight to 50% by weight, in particular from 10% by weight to 30% by weight, each based on the weight of the agent.

6. The use according to any one of claims 1 to 5, **characterized in that** the agent has a pH of 1 to 3.

7. The use according to any one of claims 1 to 6, **characterized in that** the agent additionally contains oxalic acid.

8. The use according to any one of claims 1 to 7, **characterized in that** the agent additionally contains at least one reductone.

9. The use according to any one of claims 1 to 8, **characterized in that** the agent additionally contains at least one thickening polymer.

10. A method for reductive decolouration of keratin-containing fibers, in particular human hair, in which an agent as described in one of claims 1 to 9 is applied to the keratin-containing fibers and is rinsed off again after a treatment time.

## Revendications

1. Utilisation d'un agent avec une valeur de pH de 1 à 5, contenant, dans un support, une combinaison de substances actives de
(a) au moins un composé organique répondant à la formule (I) :
**HS-X-COOM** **(I)**
dans laquelle
X représente un squelette d'hydrocarbure linéaire ou ramifié, saturé ou insaturé, qui est substitué de manière facultative avec au moins un des groupes suivants : un groupe thiol, un groupe carboxyle, un groupe carboxylate, un groupe hydroxyle, un groupe -NH2, un groupe alkyl(en C1-C6)amino, un groupe dialkyl(en C1-C6)amino, un groupe hydroxyalkyle en C1-C6 ; et
M représente un atome d'hydrogène, un groupe alkyle en C1-C8 ou un équivalent d'un cation monovalent ou polyvalent ;
et
(b) au moins un carbonate organique cyclique à titre de composé organique, pour la décoloration par réduction de fibres kératiniques, en particulier de cheveux humains.

2. Utilisation selon la revendication 1, **caractérisée en ce que** le composant (a) est choisi parmi au moins un représentant du groupe qui est formé par la L-cystéine (acide ou sel), la D-cystéine (acide ou sel), la D,L-cystéine (acide ou sel) et l'acétylcystéine.

3. Utilisation selon l'une quelconque des revendications 1 ou 2, **caractérisée en ce que** le composant (b) est choisi parmi au moins un ester cyclique d'acide carbonique répondant à la formule (II-1) 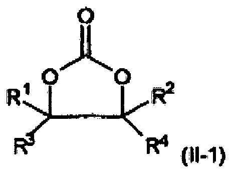 dans laquelle les résidus R1, R2, R3 et R4 représentent indépendamment l'un de l'autre un atome d'hydrogène ou des résidus organiques, en particulier un résidu alkyle, un résidu alcényle ou un résidu alkylaryle, qui peuvent en outre être substitués avec d'autres groupes, en particulier des groupes hydroxyle.

4. Utilisation selon l'une quelconque des revendications 1 à 3, **caractérisée en ce que** l'agent contient les composés du composant (a) en une quantité de 1 à 10 % en poids, en particulier de 1 à 5 % en poids, chaque fois rapportés au poids de l'agent dans sa totalité.

5. Utilisation selon l'une quelconque des revendications 1 à 4, **caractérisée en ce que** l'agent contient les composés du composant (b) en une quantité de 5 à 50 % en poids, en particulier de 10 à 30 % en poids, chaque fois rapportés au poids de l'agent.

6. Utilisation selon l'une quelconque des revendications 1 à 5, **caractérisée en ce que** l'agent possède une valeur de pH de 1 à 3.

7. Utilisation selon l'une quelconque des revendications 1 à 6, **caractérisée en ce que** l'agent contient en outre de l'acide oxalique.

8. Utilisation selon l'une quelconque des revendications 1 à 7, **caractérisée en ce que** l'agent contient en outre au moins une réductone.

9. Utilisation selon l'une quelconque des revendications 1 à 8, **caractérisée en ce que** l'agent contient au moins un polymère épaississant.

10. Procédé pour la décoloration de fibres kératiniques, en particulier de cheveux humains, par réduction, dans lequel on applique sur les fibres kératiniques un agent décrit dans l'une quelconque des revendications 1 à 9 et on l'en élimine par rinçage après l'avoir laissé agir pendant un certain temps.
